# EUROPÄISCHE PATENTSCHRIFT

(11) **EP 0 471 813 B1**
(45) Veröffentlichungstag und Bekanntmachung des Hinweises auf die Patenterteilung: **22.05.1996**
(21) Anmeldenummer: 91905110.2
(22) Anmeldetag: 05.03.1991
(51) Int. Cl.: C07C 43/225, C09K 19/12, C09K 19/30, C09K 19/14, C07C 25/18

(54) **4,4'-DISUBSTITUIERTE 2',3-DIFLUORBIPHENYLE UND FLÜSSIGKRISTALLINES MEDIUM**
4,4'-DISUBSTITUTED 2',3-DIFLUORBIPHENYLS AND LIQUID CRYSTALLINE MEDIUM
2',3-DIFLUORBIPHENYLES 4,4'-DISUBSTITUES ET MILIEU CRISTALLIN LIQUIDE

(30) Priorität: 13.03.1990 DE 4007863; 26.10.1990 DE 4034124
(43) Veröffentlichungstag der Anmeldung: 26.02.1992
(73) Patentinhaber: MERCK PATENT GmbH, D-64271 Darmstadt (DE)
(72) Erfinder: POETSCH, Eike, D-6109 Mühltal 6 (DE); REIFFENRATH, Volker, D-6101 Ro dorf (DE); HITTICH, Reinhard, D-6101 Modautal 1 (DE); RIEGER, Bernhard, Wacore Tamagawagakuen, Yokohama-shi Kanagawpref 227 (JP)
(86) Internationale Anmeldenummer: EP9100411
(87) Internationale Veröffentlichungsnummer: WO9113850

(56) Entgegenhaltungen:
- EP-A- 0 205 998
- EP-A- 0 256 636
- WO-A-90/08757
- DE-A- 3 906 052

## Beschreibung

Die Erfindung betrifft neue 4,4'-disubstituierte 2',3-Difluorbiphenyle der Formel I 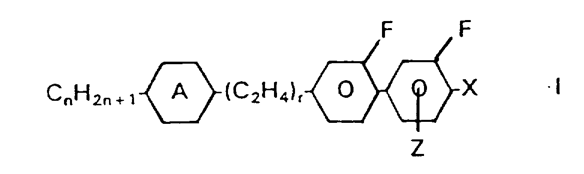 worin
- n: 1 bis 7,
- A: trans-1,4-Cyclohexylen oder 1,4-Phenylen,
- r: 0 oder 1,
- X: F, CI, -CF₃, -CHF₂, -OCF₃ oder -OCHF₂ und
- Z: H oder F
bedeuten,
mit der Maßgabe, daß im Fall
r = 1 und Z = H X -CHF₂, -OCF₃ oder -OCHF₂ ist.

In den DE-OS 30 42 391 und EP-OS 0 256 636 sind allgemeine Formeln angegeben, die die erfindungsgemäßen Verbindungen zum Teil umfassen. Es sind jedoch dort weder konkrete Derivate des 2',3-Difluorbiphenyls beschrieben noch deren vorteilhafte Wirkungen als Komponenten von flüssigkristallinen Medien angegeben.

Die Verbindungen der Formel I können wie ähnliche, z.B. aus der DE-OS 30 42 391 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hohem Klärpunkt und hoher dielektrischer Anisotropie, hervorragender Nematogenität bis zu tiefen Temperaturen, hervorragender chemischer Stabilität, ausgeprägtem ε1 bei positiver dielektrischer Anisotropie, geringer Temperaturabhängigkeit der Schwellenspannung und/oder kleiner optischer Anisotropie erhältlich. Die neuen Verbindungen zeigen außerdem eine gute Loslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, A, r, X und Z die angegebene Bedeutung, sofern nicht ausdrücklich etwas anderes vermerkt ist, Cy bedeutet 1,4-Cyclohexylen und Ph 1.4-Phenylen.

In den Verbindungen der Formel I sind die Alkylgruppen CₙH₂ₙ₊₁ vorzugsweise geradkettig. Dementsprechend bedeutet CₙH₂ₙ₊₁ Methyl, Ethyl, n-Propyl, n-Butyl, n-Pentyl, n-Hexyl oder n-Heptyl. n ist vorzugsweise 2, 3, 4 oder 5.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl). Isobutyl (= 2-Methyl-propyl), 2-Methyl-butyl, Isopentyl (= 3-Methylbutyl), 2-Methylpentyl, 3-Methylpentyl oder 2-Heptyl (= 1-Methylhexyl). 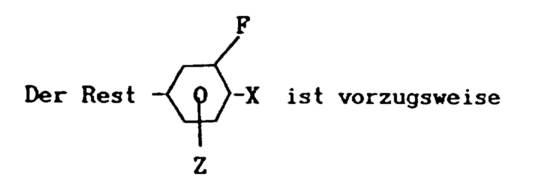 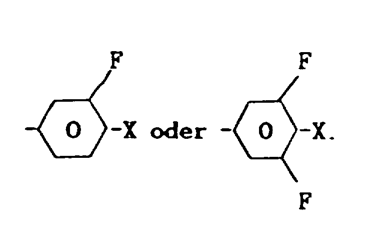 X ist vorzugsweise F, Cl, -CF₃, OCHF₂ oder -OCF₃. Besonders bevorzugt sind die Verbindungen der folgenden Teilformeln: 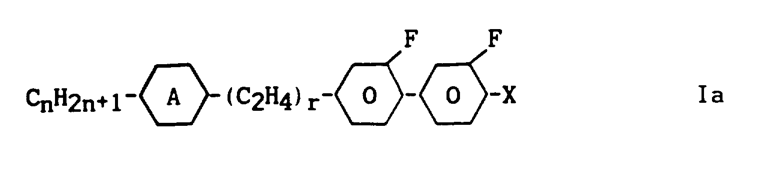 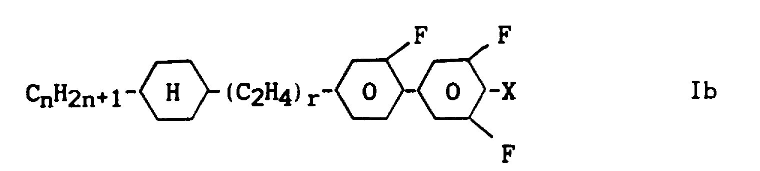 worin X F, Cl, -CF₃, -CHF₂, -OCF₃ oder -OCHF₂ ist.

Die Vorstufen der Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I können durch an sich bekannte, übergangsmetallkatalysierte Kopplungsreaktionen (vgl. E. Poetsch, Kontakte 1988 (2), S. 15) z.B. nach den folgenden Syntheseschemata hergestellt werden. Die Vorstufen sind z.T. bekannt, alle Vorstufen können nach literaturbekannten Kopplungsreaktionen hergestellt werden.

Die als Ausgangsstoffe verwendeten Brombenzolderivate sind zum Teil bekannt, zum Teil können sie ohne Schwierigkeiten nach Standardverfahren der organischen Chemie aus literaturbedingten Verbindungen hergestellt werden. Beispielsweise sind die OCF₃- oder OCHF₂-Verbindungen nach bekannten Verfahren aus den entsprechenden Phenolen bzw. die CF₃- oder CN-Verbindungen aus den entsprechenden Benzoesäuren erhältlich. Verbindungen der Formel 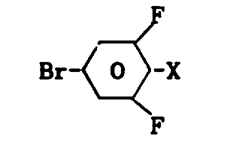 oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

### Schema 1

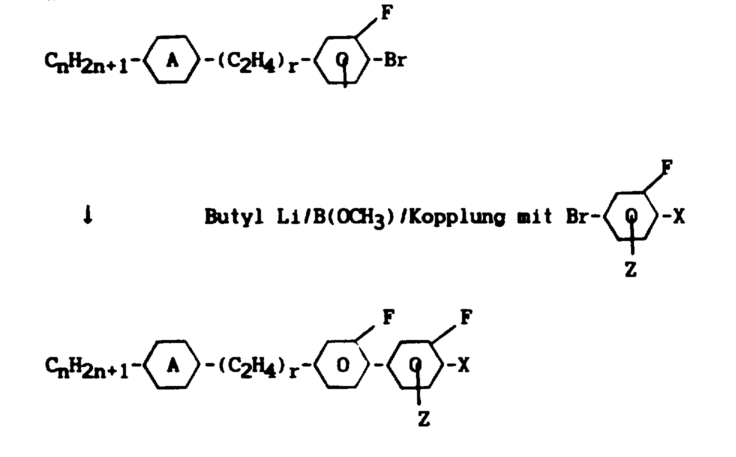

### Schema 2

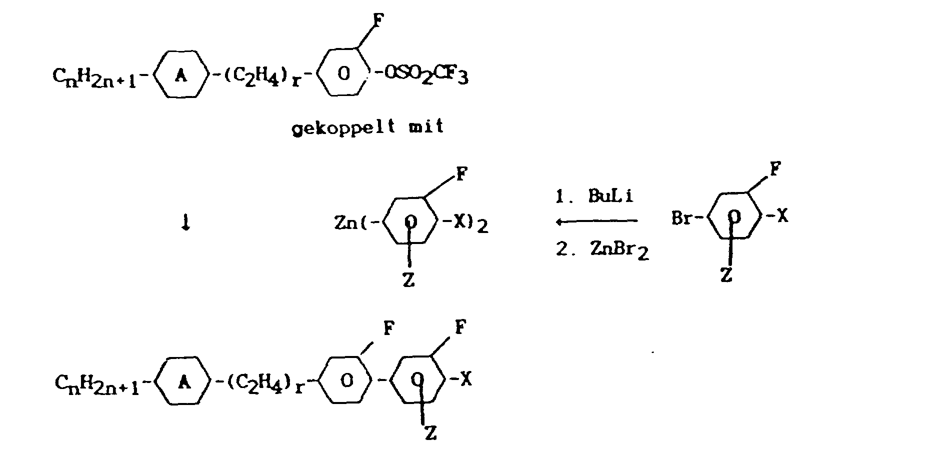

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclobexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cycloherylcyclohexylcyclohexene, 1,4-Bis-cyclohexylbenzole, 4,4'-Bis-cyclohexylbiphenyle, Phenyl-oder Cyclohexylpyrimidine, Phenyl- oder Cyclohexyipyridine, Phenyl- oder Cyclohexyldioxane, Phenyl-oder Cyclohexyl-1,3-dithiane, 1,2-Diphenyiethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-CC-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-cyclobexylen oder 1,4-Cyclobexenylen, Pyr Pyrimidin-2,5-diyl oder Pyridin-2,5-diyl, Dio 1,3-Dioxan-2,5-diyl und G 2-(trans-1 ,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxalkyl ist.

In einer anderen als Gruppe B bezeichneten kleineren Untergruppe der Verbindungen der Formeln 1, 2, 3, 4 und 5 bedeutet R" -F, -Cl oder -(O)ᵢCH₃₋₍ₖ₊₁₎FₖCl₁, wobei i 0 oder 1 und k+1 1, 2 oder 3 sind; die Verbindungen, in denen R" diese Bedeutung hat, werden mit den Teilformeln 1b, 2b, 3b, 4b oder 5b bezeichnet. Besonders bevorzugt sind solche Verbindungen der Teilformeln 1b, 2b, 3b, 4b und 5b, in denen R" die Bedeutung -F, -Cl, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN oder -NCS; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Allediese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere, vorzugsweise 1, 2, 3 oder mehrere, insbesondere 2, 3, 4 oder 5, Verbindungen ausgewählt aus der Gruppe A, und/oder eine oder mehrere, vorzugsweise 1, 2, 3 oder mehrere, insbesondere 2, 3, 4 oder 5, Verbindungen ausgewählt aus der Gruppe B und/oder eine oder mehrere, vorzugsweise 1, 2, 3 oder mehrere, insbesondere 2, 3, 4 oder 5, aus der Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise zwei, drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daB sie in allen bisher bekannt gewordenen Arten von Flüssigkriatallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- DMSO: Dimethylsulfoxid
- KOT: Kalium-tertiär-butanolat
- THF: Tetrahydrofuran
- pTSOH: p-Toluolsulfonsäure

### Beispiel 1

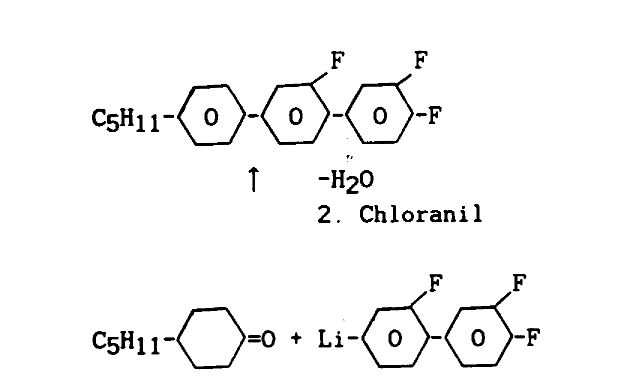

0,1 m der durch Dehydratisierung des Alkohols gewonnenen Cyclohexenverbindung (vgl. obiges Schema) werden mit 0,4 m Chloranil in 500 ml Toluol 6 h am Rückfluß gekocht. Nach extraktiver Aufarbeitung erhält man nach üblicher Aufreinigung das Zielprodukt, K 67 S_{A} 73 N 87.7 I, Δε = +11,48.

### Beispiel 2 bis 34:

Analog Beispiel 1 erhält man die folgenden Verbindungen der Formel I (r = O/A = Ph)

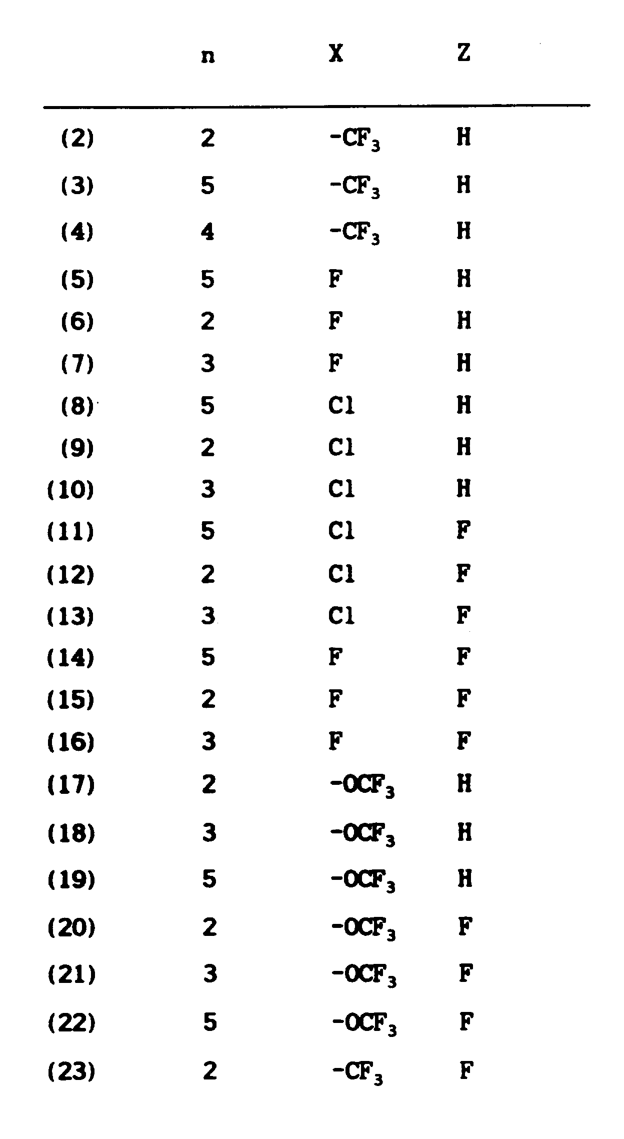

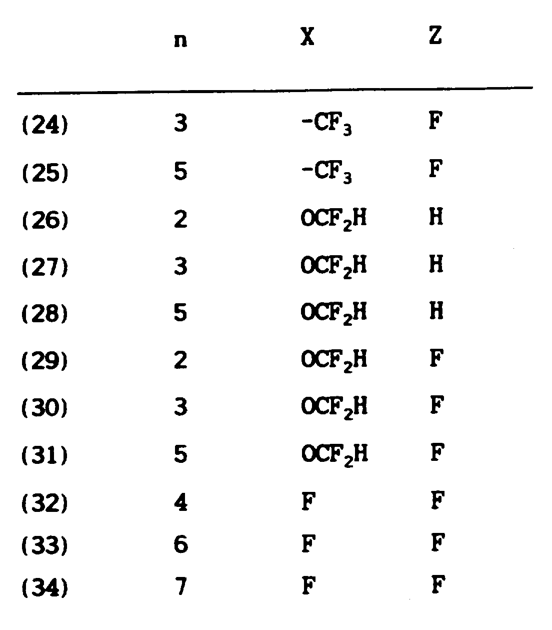

### Beispiel 35:

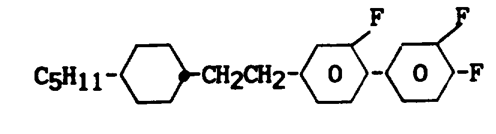

0,05 m 3,4-Difluorbrombenzol, 0,05 m p-[2-(trans-4-n-Pentylcyclohexyl)-ethyl]-o-fluorphenylboronsäure und 1 g Tetrakistriphenylphosphinpalladium -O Katalysator werden in 100 ml Toluol und 40 ml Ethanol gelöst und mit 50 ml 2 m-Na₂CO₃-Lsg. versetzt. Man kocht 4 h am Rückfluß und arbeitet danach extraktiv auf. Aus dem Rohmaterial erhält man durch Chromatographie und Kristallisation das saubere Produkt.

### Beispiel 36-83:

Analog Beispiel 35 erhält man die folgenden Verbindungen der Formel I (r = 1)

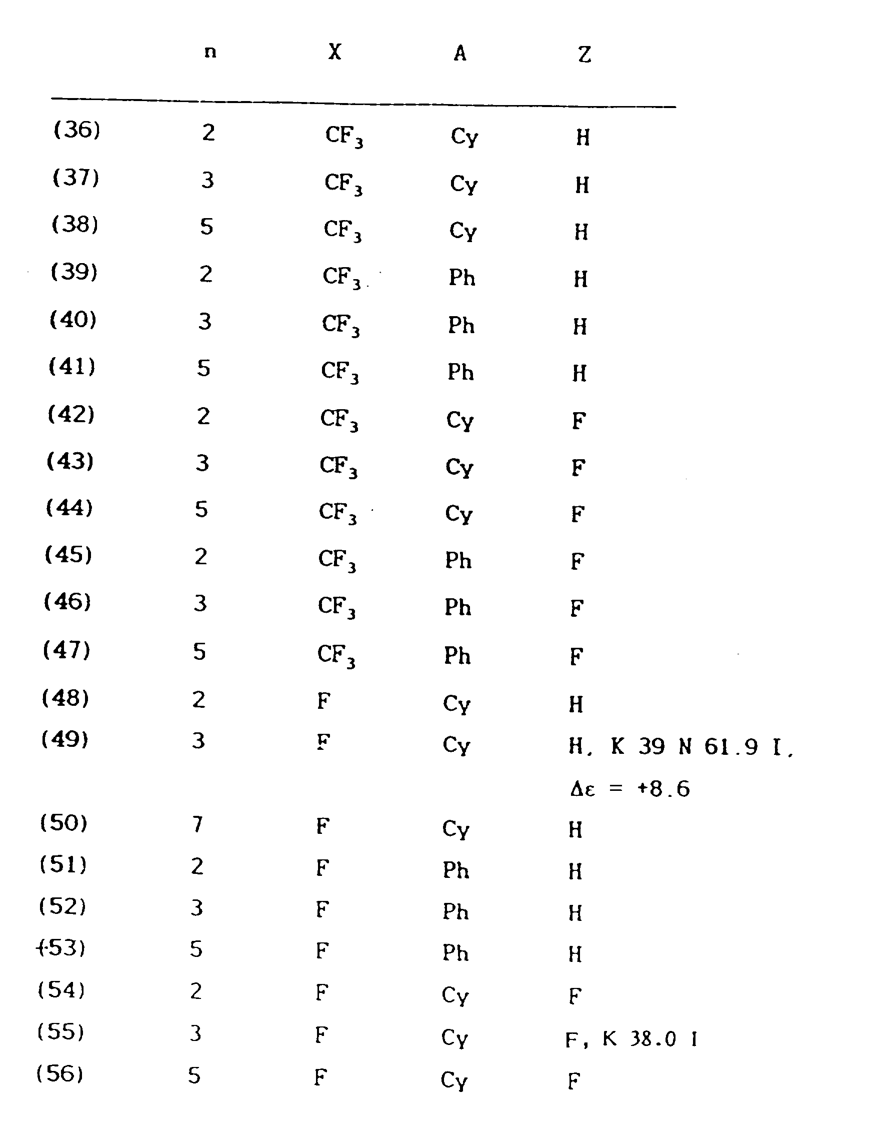

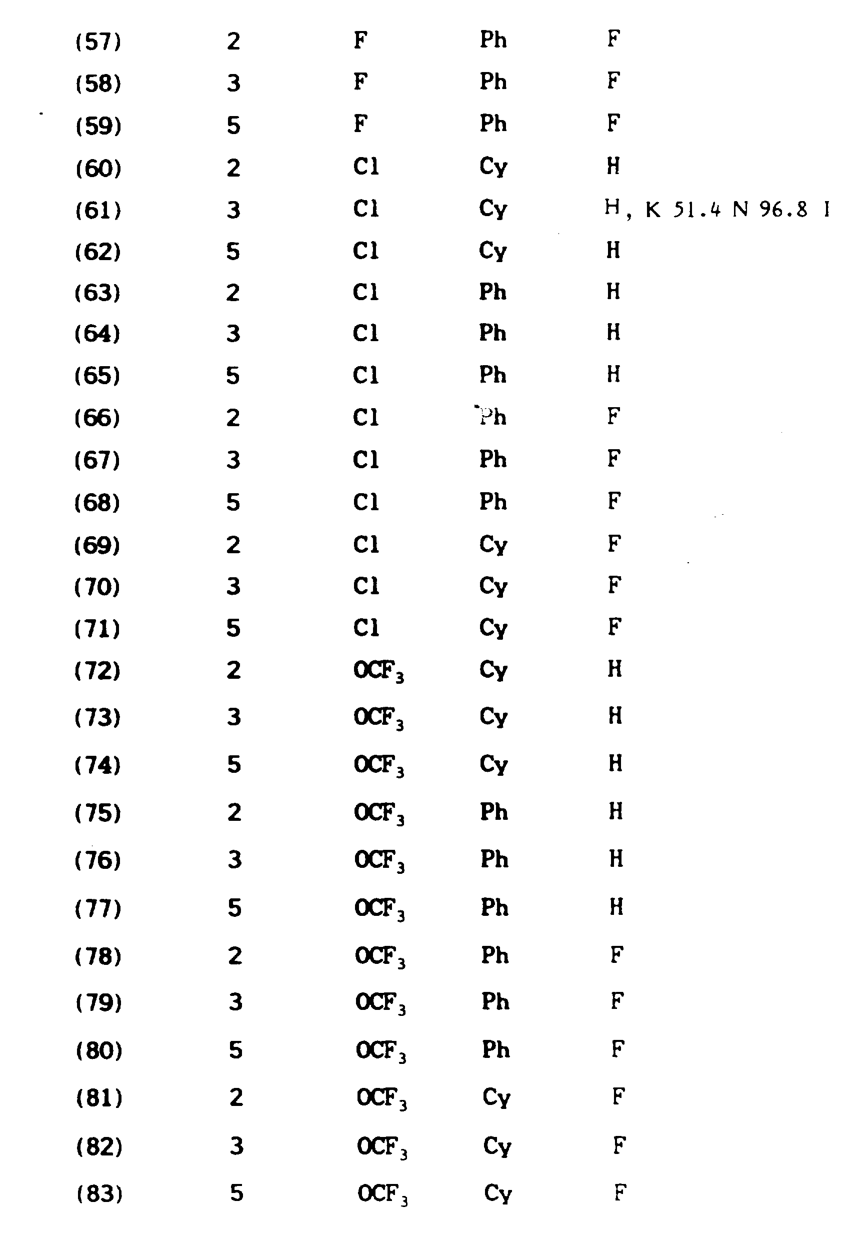

### Beispiel 84

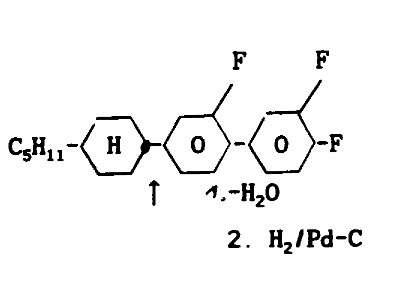

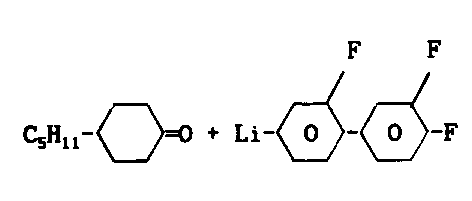

0,1 m der durch Dehydratisierung des Alkohols gewonnenen Cyclohexenverbindung (vgl. obiges Schema) werden mit 5 g Pd-C (5%ig in 500 ml Toluol) bis zur Sättigung hydriert. Nach extraktiver Aufarbeitung erhält man nach üblicher Aufreinigung das Zielprodukt.

### Beispiel 85 bis 114

Analog Beispiel 84 erhält man die folgenden Verbindungen der Formel I (r = O/A = Cy)

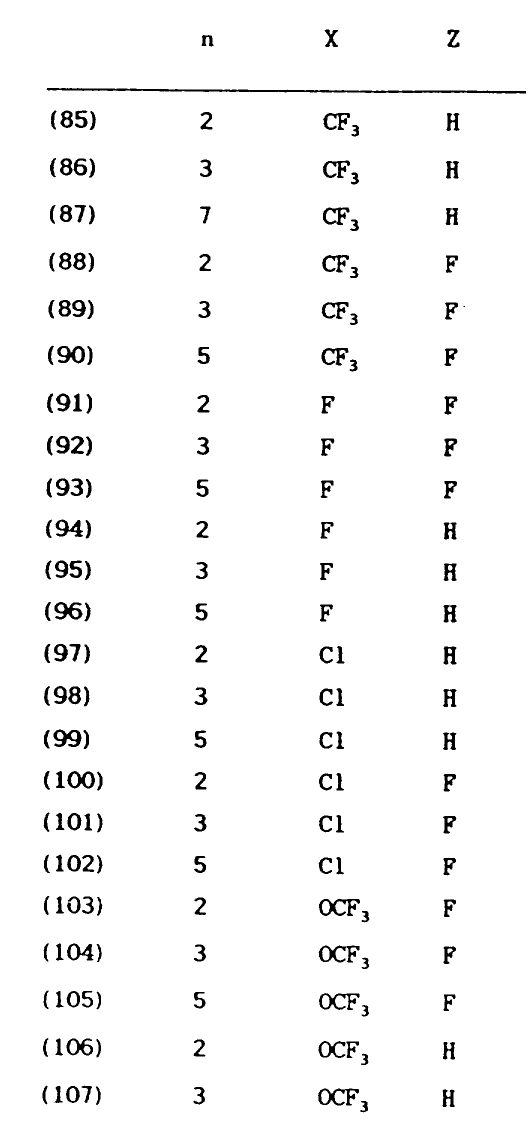

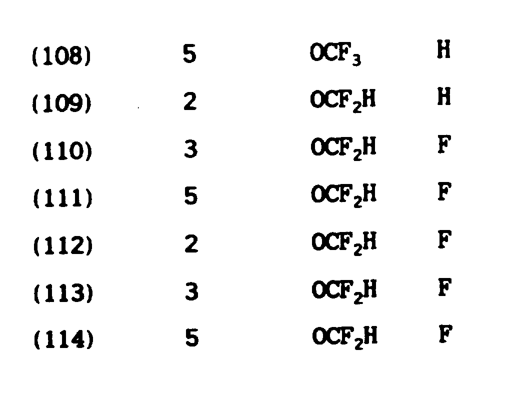

## Patentansprüche

1. 4,4'-Disubstituierte 2',3-Difluorbiphenyle der Formel I 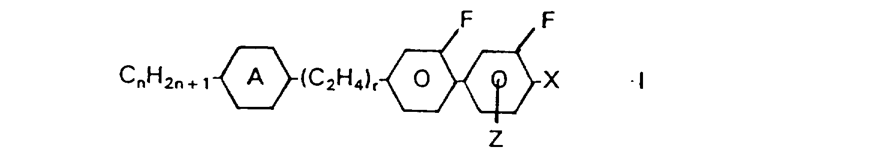 worin
n 1 bis 7,
A trans-1,4-Cyclohexylen oder 1,4-Phenylen,
r 0 oder 1,
X F, CI, -CF₃, -CHF₂, -OCF₃ oder -OCHF₂ und
Z H oder F
bedeuten,
mit der Maßgabe, daß im Fall
r = 1 und Z = H X -CHF₂, -OCF₃ oder -OCHF₂ ist.

2. Biphenyle nach Anspruch 1, gekennzeichnet durch die Formel Ia 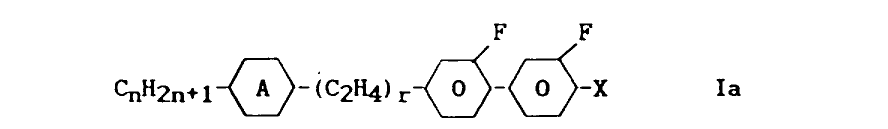 worin X F, Cl, -CF₃, -CHF₂, -OCF₃ oder -OCHF₂ ist.

3. Biphenyle nach Anspruch 1, gekennzeichnet durch die Formel Ib 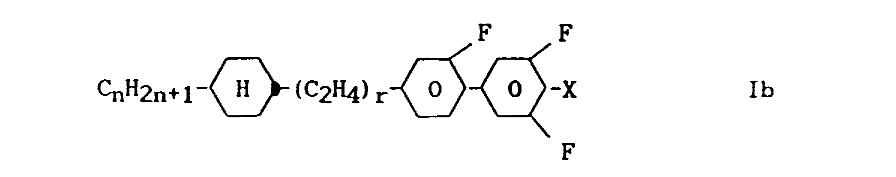 worin X F, Cl, -CF₃, -CHF₂, -OCF₃ oder -OCHF₂ ist.

4. Verwendung der Biphenyle der Formel I nach Anspruch 1 als Komponenten flüssigkristalliner Medien für elektrooptische Anzeigen.

5. Flüssigkristallines Medium für elektrooptischte Anzeigen mit mindestens zwei flüssigkristallinen Komponenten, dadurch gekennzeichnet, daß mindestens eine Komponente ein Biphenyl der Formel I nach Anspruch 1 ist.

6. Elektrooptische Anzeige auf der Basis einer Flüssigkristallzelle, dadurch gekennzeichnet, daß die Flüssigkristallzelle ein Medium nach Anspruch 5 enthält.

## Claims

1. 4,4'-disubstituted 2',3-difluorobiphenyls of the formula I 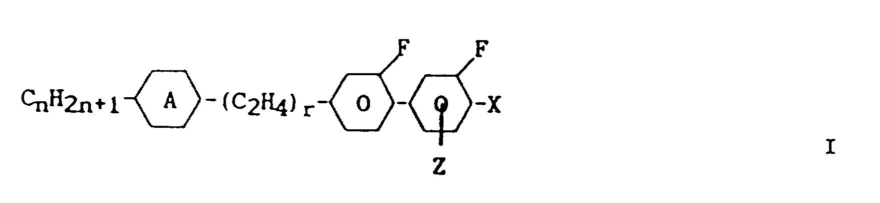 in which n is 1 to 7, A is trans-1,4-cyclohexylene or 1,4-phenylene, r is 0 or 1, X is F, Cl, -CF₃, -CHF₂, -OCF₃ or -OCHF₂ and Z is H or F
with the proviso that if V=1 and Z=H, X denotes -CHF₂, -OCF₃ or OCHF₂.

2. Biphenyls according to claim 1, characterized by the formula Ia 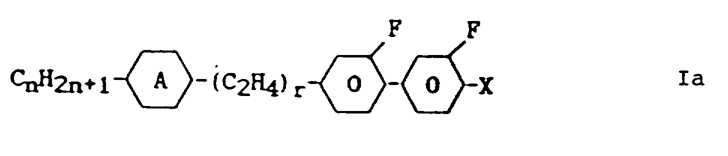 in which X is F, Cl, -CF₃, -CHF₂, -OCF₃ or -OCHF₂.

3. Biphenyls according to claim 1, characterized by the formula Ib 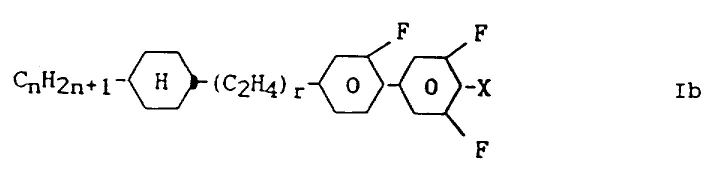 in which X is F, Cl, -CF₃, -CHF₂, -OCF₃ or -OCHF₂.

4. Use of the biphenyls of the formula I according to claim 1 as components of liquid-crystalline media for electrooptical displays.

5. Liquid-crystalline medium for electrooptical displays having at least two liquid-crystalline components, characterized in that at least one component is a biphenyl of the formula I according to claim 1.

6. Electrooptical display based on a liquid-crystal cell, characterized in that the liquid-crystal cell contains a medium according to claim 5.

## Revendications

1. 2',3-difluorobiphényles 4,4'-disubstitués de formule I 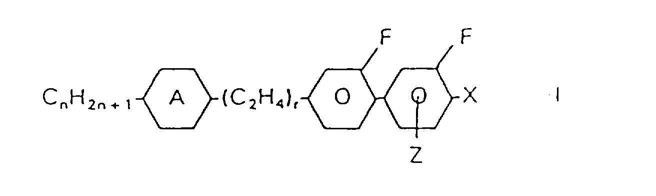 dans laquelle
n vaut de 1 à 7,
A représente un trans-1,4-cyclohexylène ou un 1,4-phénylène,
r vaut 0 ou 1,
X représente F, Cl, -CF₃, -CHF₂, -OCF₃ ou -OCHF₂ et
Z représente H ou F,
sous réserve qu'au cas où r = 1 et Z = H, X représente -CHF₂, -OCF₃ ou -OCHF₂.

2. Biphényles selon la revendication 1, caractérisés par la formule Ia 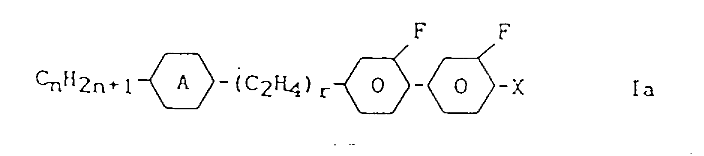 dans laquelle X représente F, Cl, -CF₃, -CHF₂, -OCF₃ ou -OCHF₂.

3. Biphényles selon la revendication 1, caractérisés par la formule Ib 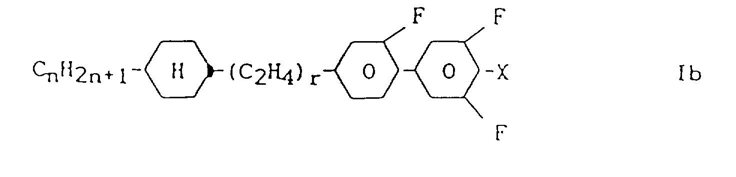 dans laquelle X représente F, Cl, -CF₃, -CHF₂, -OCF₃ ou -OCHF₂.

4. Application des biphényles de formule I selon la revendication 1 comme composants de milieux à cristaux liquides pour affichages électro-optiques.

5. Milieu à cristaux liquides pour affichages électro-optiques avec au moins deux composants à cristaux liquides, caractérisé en ce qu'au moins un composant est un biphényle de formule I selon la revendication 1.

6. Affichage électro-optique à base de cellule à cristaux liquides, caractérisé en ce que la cellule à cristaux liquides contient un milieu selon la revendication 5.
